# EUROPEAN PATENT SPECIFICATION

(11) **EP 3 344 343 B1**
(45) Date of publication and mention of the grant of the patent: **17.02.2021**
(21) Application number: 16766745.0
(22) Date of filing: 02.09.2016
(51) Int. Cl.: A61Q 5/00, A61K 8/892, A61K 8/893, A61K 8/898

(54) **SILICONES FOR PROTECTING HAIR AGAINST HEAT**
SILIKONVERBINDUNGEN ZUM SCHUTZ DER HAARE VOR HITZE
SILICONES POUR PROTEGER LES CHEVEUX DE LA CHALEUR

(30) Priority: 02.09.2015 US 201562213361 P; 19.05.2016 US 201662338796 P
(43) Date of publication of application: 11.07.2018
(73) Proprietor: Dow Silicones Corporation, Midland, MI 48686-0994 (US)
(72) Inventor: BRUNI, Antonio, 9451 Kerksken (BE); DUPONT, Anne, 1400 Nivelles (BE); JOHNSON, Bethany, Midland, MI 48642 (US); MARCHIORETTO, Sabrina, 1180 Bruxelles (Uccle) (BE); NGUYEN, Kimmai, Thi, Midland, MI 48640 (US); PERRIN, Ludovic, 7160 Chap.-Lez-Herlaimont (BE)
(74) Representative: Murgitroyd & Company
(86) International application number: PCT/US2016/050025
(87) International publication number: WO 2017/040876

(56) References cited:
- WO-A1-2008/103219
- WO-A1-2012/027144
- WO-A1-2012/027145
- WO-A2-2010/047993

## Description

### Technical Field

This disclosure relates to compositions and methods for protecting hair and other fibers against heat by applying to the hair or fibers the compositions containing silicone and silicone polyether copolymers with groups having amino, hydroxy and/or alkoxy functionalities. The compositions may comprise emulsions of such silicone and silicone polyether copolymers.

### Background

Hair is exposed to heat treatment in drying and styling processes. For example, hair is exposed to hot air from blow dryers and to the heated cylinders and platforms of hot curling and straightening irons. This heat can cause hair to become damaged and lose strength. The results of this damage can cause the appearance of hair to degrade and the hair to become brittle and subject to breakage.

Compositions have been developed and marketed to protect the hair from the effects of heat treatment. Many of these compositions function by forming a coating of the hair to decrease the penetration of the heat directly to the hair. However, there remain unmet needs for protecting hair against heat. For example, existing compositions can be improved to better prevent hair breakage on combing.

### Summary

The present invention relates to a method for protecting hair from heat damage, the method comprising applying to hair a composition comprising a silicone polyether copolymer having an average formula

A-R₂SiO(R₂SiO)ₓSiR₂-[[R¹O(CₘH₂ₘO)_{y}R¹][R₂SiO(R₂SiO)ₓ]R₂Si]*ₙ*-A

wherein
A is an aminofunctional endblocking group of the formula R^{A}CH₂CH(OH)CH₂OR²-
wherein R^{A} is an aminofunctional group,
x is ≥ 0, m is from 2 to 4 inclusive, y is ≥ 4, *n* is ≥ 1,
R is independently a monovalent hydrocarbon group containing 1 to 30 carbons, a hydroxy group or an alkoxy group,
R¹ is a divalent hydrocarbon containing 2 to 30 carbons,
R² is a divalent hydrocarbon linking group containing 2 to 20,
wherein the composition provides protection.

The method of the invention provides treated hair that is protected from damage caused by heat. Also disclosed is that compositions may be formulated into hair care products.

Preferred embodiments of the invention are set forth in the dependent claims.

Associated methods are also described herein to aid in the understanding of the invention, but these do not form part of the claimed invention. Examples or embodiments described herein which do not fall under the definition of the claims do not form part of the present invention.

### Detailed Description

There is provided a method for protecting hair from heat, the method comprising applying to hair a composition comprising a silicone polyether copolymer having an average formula

A-R₂SiO(R₂SiO)ₓSiR₂-[[R¹O(CₘH₂ₘO)_{y}R¹][R₂SiO(R₂SiO)ₓ]R₂Si]*ₙ*-A

wherein
A is an aminofunctional endblocking group of the formula R^{A}CH₂CH(OH)CH₂OR²-
wherein R^{A} is an aminofunctional group,
x is ≥ 0, m is from 2 to 4 inclusive, y is ≥ 4, *n* is ≥ 1,
R is independently a monovalent hydrocarbon group containing 1 to 30 carbons, a hydroxy group or an alkoxy group,
R¹ is a divalent hydrocarbon containing 2 to 30 carbons,
R² is a divalent hydrocarbon linking group containing 2 to 20 carbon atoms,
wherein the composition provides protection.

The silicone polyether copolymers of the present disclosure are block copolymers having repeating units of a divalent organic group containing at least one polyether group as one block, and a diorganopolysiloxane as the other block, represented as [[R¹O(CₘH₂ₘO)_{y}R¹][R₂SiO(R₂SiO)_{x]}R₂Si]*ₙ*. The subscript n represents on average the number repeating units in the copolymer, and *n* is ≥ 1, alternatively *n* ranges from 1 to 50.

The divalent organic group in the silicone polyether copolymers of the present disclosure comprises at least one polyether group. As used herein, "polyether" designates a polyoxyalkylene group. The polyoxyalkylene group may be represented by the formula (CₘH₂ₘO)_{y} wherein m is from 2 to 4 inclusive, and y is greater than 4, alternatively y may range from 5 to 60, or alternatively from 5 to 30. The polyoxyalkylene group may comprise oxyethylene units (C₂H₄O), oxypropylene units (C₃H₆O), oxybutylene units (C₄H₈O), or mixtures thereof. Typically, the polyoxyalkylene group comprises oxyethylene units (C₂H₄O) or mixtures of oxyethylene units and oxypropylene units.

The "silicone" group in the silicone polyether copolymers of the present disclosure is a diorganopolysiloxane. The diorganopolysiloxane may be a predominately linear siloxane polymer having the formula (R₂SiO)ₓ, wherein R is independently selected from a monovalent hydrocarbon group, x is ≥ 1, alternatively x may range from 2 to 100, or from 2 to 50. The hydrocarbon groups represented by R in the siloxane polymer are free of aliphatic unsaturation. These organic groups may be independently selected from monovalent hydrocarbon and monovalent halogenated hydrocarbon groups free of aliphatic unsaturation. These monovalent groups preferably have from 1 to 30 carbon atoms, although alternatively 1 to 10 carbon atoms is also disclosed, and are exemplified by alkyl groups such as methyl, ethyl, propyl, butyl, pentyl, hexyl, heptyl, octyl, undecyl, and octadecyl; cycloalkyl such as cyclohexyl; aryl such as phenyl, tolyl, xylyl, benzyl, and 2-phenylethyl; and halogenated hydrocarbon groups such as 3,3,3-trifluoropropyl, 3-chloropropyl, and dichlorophenyl. Typically, the diorganopolysiloxane is a predominately linear polydimethylsiloxane having the formula (Me₂SiO)ₓ, where x is as defined above.

At least one end of each polyether block is linked to an organopolysiloxane block by a divalent hydrocarbon group, designated R¹. This linkage is determined by the reaction employed to prepare the (AB)ₙ block silicone polyether copolymer. The divalent hydrocarbon group R¹ may be independently selected from divalent hydrocarbon groups containing 2 to 30 carbons. Representative examples of such divalent hydrocarbon groups include; ethylene, propylene, butylene, isobutylene, pentylene, hexylene, heptylene and octylene. Representative, examples of such divalent organofunctional hydrocarbons groups include acrylate and methacrylate. Typically, R¹ is isobutylene (-CH₂CH(CH₃)CH₂-).

The aminofunctional endblocking group A may have the formula R^{A}CH₂CH(OH)CH₂OR²- where R^{A} is a monovalent amine functional group and R² is a divalent hydrocarbon linking group preferably containing 2 to 20, although alternatively 2 to 6 carbon atoms is also disclosed, such as a divalent alkylene like ethylene, propylene, butylene, isobutylene, pentylene, or hexylene. Typically, R² is propylene -CH₂CH₂CH₂-. The monovalent amine functional group R^{A} may be any amine functional organic group. The nitrogen atom of the amine functional group is bonded to the methylene group of the-CH₂CH(OH)CH₂OR²- endblocking group. The amine functional group may be any secondary, tertiary, or quaternary amine, but typically are tertiary amines. The amine functional group may be include other organic functional groups, such as amino, hydroxy, epoxy, ether, amido, and carboxyl groups. Thus, R^{A} may have the formula (R³)₂N-, H(R³)N-, or (R³)₃N-, wherein R³ is independently a monovalent organic containing 1 to 30 carbon atoms. Alternatively, R³ is independently a monovalent hydrocarbon group containing 1 to 30 carbon atoms, such as alkyl groups containing 1 to 30 carbons like methyl, ethyl, propyl, butyl, and similar homologs. Representative, non limiting examples include; (CH₃)HN-, (CH₃)₂N-, (CH₃CH₂)HN-, (CH₃CH₂)₂N-, (CH₃CH₂)₃N-, (HOCH₂CH₂)₂N-, and [CH₂CH(OH)CH₃]₂N-. The amine functional group may include cyclic amines such as; pyrrolidine; piperidine; piperazine; morpholine; 3-pyrrolidinol; 2,5-dimethylpyrrolidine; 1-methylpiperazine; 4-hydroxypiperidine; N-(2-hydroxyethyl)piperazine, 2,6-dimethylpiperidine; 1-ethylpiperazine; 1-amine-4-methylpiperazine; and isoindoline.

The aminofunctional endblocking group A may have the formula, 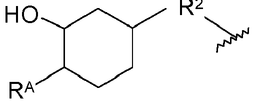 where R^{A} and R² are the same as described above.

Representative average formulas of the amine terminal silicone polyethers of the present disclosure are shown below.

Et₂NCH₂CH(OH)CH₂O(CH₂)₃(Me₂SiO)ₓ-Me₂Si[[(CH₂)₃O(EO)_{y'}(CH₂)₃][(Me₂SiO)ₓ Me₂Si]]*ₙ*(CH₂)₃OCH₂CH(OH)CH₂NEt₂,

Et₂NCH₂CH(OH)CH₂O(CH₂)₃(Me₂SiO)ₓ-Me₂Si[[(CH₂)₃O[(EO)_{y'}(PO)_{y"}] (CH₂)₃][(Me₂SiO)ₓMe₂Si]]*ₙ*(CH₂)₃OCH₂CH(OH)CH₂NEt_{2,}

Et₂ NCH₂CH(OH)CH₂O(CH₂)₃(Me₂SiO)ₓMe₂Si[[CH₂CH(Me)CH₂O[(EO)_{y'}]CH₂CH(Me)CH₂][(Me₂SiO)ₓMe₂Si]]*ₙ*(CH₂)₃OCH₂CH(OH)CH₂NEt₂,

Et₂NCH₂CH(OH)CH₂O(CH₂)₃(Me₂SiO)ₓ-Me₂Si[[CH₂CH(Me)CH₂O[(EO)_{y'}(PO)_{y"} ]CH₂CH(Me)CH₂][(Me₂SiO)ₓMe₂Si]]*ₙ*(CH₂)₃OCH₂CH(OH)CH₂NEt₂,

wherein n and x are as defined above,
y' is ≥ 0, alternatively y' is 0 to 60,
y" is ≥ 0, alternatively y" is 0 to 60,
with the proviso that y' + y" ≥ 4
Me is methyl, Et is ethyl,
EO is CH₂CH₂O, and
PO is CH₂ CH(Me)O or CH₂CH₂CH₂O.

The aminofunctional endblocked silicone polyether block copolymers may be prepared by:
I) reacting;
   A) a polyoxyalkylene having an unsaturated hydrocarbon group at each molecular terminal
   B) a SiH terminated organopolysiloxane,
   C) a hydrosilylation catalyst,
   D) an optional solvent, where the molar ratio of B/A is greater than one,
II) further reacting the product of step I with;
   E) an epoxide having at least one aliphatic unsaturated hydrocarbon group to form an epoxide terminal silicone polyether block copolymer,
III) reacting the epoxide terminal silicone polyether block copolymer with
   F) an amine compound to form the amine terminal silicone polyether block copolymer.

The silicone compositions and amine terminal silicone polyether block copolymers of the present disclosure may be an ingredient in an emulsion composition. As used herein, "emulsion" is meant to encompass water continuous emulsions (for example an oil in water type emulsion, or a silicone in water emulsion), oil or silicone continuous emulsions (water in oil emulsions or water in silicone emulsions), or multiple emulsions (water/oil/water, oil/water/oil types, water/silicone/water, or silicone/water/silicone). The amine terminal silicone polyether block copolymers of the present disclosure may be added to any type of emulsion by common mixing techniques. The addition of the amine or quat terminal silicone polyether block copolymers may occur either during the preparation of the emulsion, or subsequently post added to a pre-formed emulsion. There are no special requirements or conditions needed to effect the mixing of amine terminal silicone polyether block copolymers of the present disclosure and the emulsion. Mixing techniques can be simple stirring, homogenizing, sonalating, and other mixing techniques known in the art to effect the formation of emulsions. The mixing can be conducted in a batch, semi-continuous, or continuous process.

The amount of silicone and amine terminal silicone polyether block copolymers of the present disclosure added to the emulsion can vary and is not limited, however the amounts typically may range from a silicone or silicone polyether copolymer/emulsion weight ratio of 0.1/99 to 99/0.1, alternatively 1/99 to 99/1.

The emulsions used may be w/o, w/s, or multiple phase emulsions using silicone emulsifiers. Typically the water-in-silicone emulsifier in such formulation is non-ionic and is selected from polyoxyalkylene-substituted silicones, silicone alkanolamides, silicone esters and silicone glycosides. Silicone-based surfactants may be used to form such emulsions and are well known in the art, and have been described for example in US 4,122,029 (Gee et al.), US 5,387,417 (Rentsch), and US 5,811,487 (Schulz et al).

The emulsions containing the silicone may contain anionic surfactants, cationic surfactants, and nonionic surfactants. The anionic surfactants include sulfonic acids and their salt derivatives. Some examples of anionic surfactants are alkali metal sulfosuccinates; sulfonated glyceryl esters of fatty acids such as sulfonated monoglycerides of coconut oil acids; salts of sulfonated monovalent alcohol esters such as sodium oleyl isothionate; amides of amino sulfonic acids such as the sodium salt of oleyl methyl tauride; sulfonated products of fatty acid nitriles such as palmitonitrile sulfonate; sulfonated aromatic hydrocarbons such as sodium alpha-naphthalene monosulfonate; condensation products of naphthalene sulfonic acids with formaldehyde; sodium octahydro anthracene sulfonate; alkali metal alkyl sulfates; ether sulfates having alkyl groups of eight or more carbon atoms such as sodium lauryl ether sulfate; and alkylaryl sulfonates having one or more alkyl groups of eight or more carbon atoms such as neutral salts of hexadecylbenzene sulfonic acid and C₂₀ alkylbenzene sulfonic acid.

Commercial anionic surfactants which can be used include the sodium salt of dodecylbenzene sulfonic acid sold under the trademark SIPONATE® DS-10 by Alcolac Inc., Baltimore, Maryland; sodium n-hexadecyl diphenyloxide disulfonate sold under the trademark DOWFAX® 8390 by The Dow Chemical Company, Midland, Michigan; the sodium salt of a secondary alkane sulfonate sold under the trademark HOSTAPUR® SAS 60 by Clariant Corporation, Charlotte, North Carolina; N-acyl taurates such as sodium N-lauroyl methyl taurate sold under the trademark NIKKOL LMT® by Nikko Chemicals Company, Ltd., Tokyo, Japan; and linear alkyl benzene sulfonic acids sold under the trademark BlO-SOFT® S-100 by the Stepan Company, Northfield, Illinois. Compositions of the latter type such as dodecylbenzene sulfonic acid, although a catalyst as noted above, can also function as the anionic surfactant when neutralized.

Cationic surfactants useful herein include compounds containing quaternary ammonium hydrophilic moieties in the molecule which are positively charged, such as quaternary ammonium salts represented by R3R4R5R6N⁺X⁻ where R3 to R6 are alkyl groups containing

1-30 carbon atoms, or alkyl groups derived from tallow, coconut oil, or soy; and X is halogen, i.e., chlorine or bromine. Dialkyl dimethyl ammonium salts can be used and are represented by R7R8N⁺(CH₃)₂X⁻ where R7 and R8 are alkyl groups containing 12-30 carbon atoms or alkyl groups derived from tallow, coconut oil, or soy; and X is halogen. Monoalkyl trimethyl ammonium salts can be used and are represented by R⁹N⁺(CH₃)₃X⁻ where R⁹ is an alkyl group containing 2-30 carbon atoms, alternatively 13-15 carbon atoms or an alkyl group derived from tallow, coconut oil, or soy; and X is halogen.

Representative quaternary ammonium salts are dodecyltrimethyl ammonium chloride/lauryltrimethyl ammonium chloride (LTAC), cetyltrimethyl ammonium chloride (CTAC), didodecyldimethyl ammonium bromide, dihexadecyldimethyl ammonium chloride, dihexadecyldimethyl ammonium bromide, dioctadecyldimethyl ammonium chloride, dieicosyldimethyl ammonium chloride, didocosyldimethyl ammonium chloride, dicoconutdimethyl ammonium chloride, ditallowdimethyl ammonium chloride, and ditallowdimethyl ammonium bromide. These quaternary ammonium salts are commercially available under trademarks such as ADOGEN®, ARQUAD®, TOMAH®, and VARIQUAT®.

Commercially available nonionic surfactants which can be used include compositions such as 2,6,8-trimethyl-4-nonyloxy polyethylene oxyethanols (6EO) and (10EO) sold under the trademarks TERGITOL® TMN-6 and TERGITOL® TMN-10; alkyleneoxy polyethylene oxyethanol (C₁₁₋₁₅ secondary alcohol ethoxylates 7EO, 9EO, and 15EO) sold under the trademarks TERGITOL® 15-S-7, TERGITOL® 15-S-9, TERGITOL® 15-S-15; other C₁₁₋₁₅ secondary alcohol ethoxylates sold under the trademarks TERGITOL® 15-S-12, 15-S-20, 15-S-30, 15-S-40; and octylphenoxy polyethoxy ethanol (40EO) sold under the trademark TRITON® X-405. All of these surfactants are sold by Union Carbide Corporation, Danbury, Connecticut. Other useful commercial nonionic surfactants are nonylphenoxy polyethoxy ethanol (10EO) sold under the trademark MAKON® 10 by Stepan Company, Northfield, Illinois; polyoxyethylene 23 lauryl ether (Laureth-23) sold commercially under the trademark BRIJ® 35L by ICI Surfactants, Wilmington, Delaware; and RENEX® 30, a polyoxyethylene ether alcohol sold by ICI Surfactants, Wilmington, Delaware.

Protective colloids, i.e., colloidal stabilizers, may be used, if desired, to enhance stability or to provide a specific rheological characteristic to the emulsion. As used herein, the terms *protective colloid* and/or *colloidal stabilizer* mean a nonionic molecule that is an effective agent for protecting charged colloidal particles in an aqueous media against flocculation. These compositions typically have a weight average molecular weight between 1,000-300,000 and are typically more hydrophilic than the composition of the first emulsion polymer, as measured by weight-averaged solubility parameters. Colloidal stabilizers which can be used include hydroxyethyl cellulose having a weight average molecular weight between 50,000-150,000; N-vinyl pyrrolidone; polyvinyl alcohol having a weight average molecular weight between 10,000-200,000; partially acetylated polyvinyl alcohol; carboxymethyl cellulose; gums such as gum arabic; starches; proteins; and mixtures thereof. Preferred colloidal stabilizers are hydroxethyl cellulose and polyvinyl alcohol.

Since emulsions are susceptible to microbiological contamination a preservative can be added. Representative preservatives, which can be used include formaldehyde; 1,3-dimethylol-5,5-dimethyl hydantoin, i.e., DMDM Hydantoin; 5-bromo-5-nitro-1,3-dioxane; methyl or propyl paraben; sorbic acid; imidazolidinyl urea; and KATHON® CG (5-chloro-2-methyl-4-isothiazolin-3-one).

Generally, the silicone emulsions contain a siloxane polymer concentration of 10 to 70 percent by weight based on the weight of the total emulsion, preferably 20 to 60 percent by weight. While emulsions containing less than 10 percent siloxane polymer content can be made, such emulsions hold little or no economic value. The surfactant is generally present at 0.05-30 percent by weight based on the weight of the total emulsion, preferably 0.1 to 20 percent by weight. Water and optional ingredients constitute the balance of the emulsion to 100 percent.

Compositions comprising the silicone may be formulated into hair care products such as hair shampoo, hair conditioner, leave-on hair conditioner, hair spray, hair gel, hair colorants, hair relaxers, hair mousse and other products for application to the hair, hair shampoos, hair conditioners, hair colorants, hair relaxants, hair sprays, mousses, gels, permanents. The compositions disclosed herein may be in the form of a cream, a gel, a powder, a paste, or a freely pourable liquid. Generally, such compositions can generally be prepared at room temperature if no solid materials at room temperature are presents in the compositions, using simple propeller mixers, Brookfield counter-rotating mixers, or homogenizing mixers. No special equipment or processing conditions are typically required. Depending on the type of form made, the method of preparation will be different, but such methods are well known in the art.

The compositions disclosed herein can be used by the standard methods, such as applying them to the human body, e.g. hair, using applicators, brushes, applying by hand, pouring them and/or possibly spraying, rubbing or massaging the composition onto the hair. These steps can be repeated as many times as desired to achieve the desired benefit.

The use of the compositions disclosed herein on hair may use a conventional manner for conditioning hair. An effective amount of the composition for conditioning hair is applied to the hair. Such effective amounts generally range from about 0.5 g to about 50 g, preferably from about 1 g to about 20 g. Application to the hair typically includes working the composition through the hair such that most or all of the hair is contacted with the composition. This method for conditioning the hair comprises the steps of applying an effective amount of the hair care composition to the hair, and then working the composition through the hair. These steps can be repeated as many times as desired to achieve the desired conditioning benefit.

In one example, the compositions are applied to the hair prior to subjecting the hair to heat treatment, alternatively applied to the hair up to 8 hours, alternatively up to 4 hours, alternatively up to 30 minutes, alternatively up to 15 minutes, alternatively up to 5 minutes before the hair is heat treated.

In one example, the hair is treated with heat after the composition is applied to the hair. The hair is treated with heat by subjecting the hair to temperatures up to 250 °C, alternatively from 37 to 250 °C, alternatively from 50 to 200 °C, alternatively from 95 to 200 °C.

The hair is subjected to the temperatures by blowing air onto or through the hair at the temperature, alternatively by contacting the hair with a cylinder or platform that has been heated to the temperature. The hair is typically subjected to heat or heat treated using a hair drying or curling or straightening iron.

The hair is subjected to heat for at least 1 second, alternatively from 1 second to 10 minutes, alternatively from 1 second to 1 minute, alternatively from 1 second to 20 seconds.

Not wishing to be bound by theory, it is believed that the heat causes the silicone molecules to undergo an intermolecular reactions with other silicone molecules, the hair, or both other silicone molecules and the hair.

A composition is disclosed for protecting hair from heat, comprising: applying to hair a composition comprising a silicone polyether copolymer having an average formula

A-R₂SiO(R₂SiO)ₓSiR₂-[[R¹O(CₘH₂ₘO)_{y}R¹][R₂SiO(R₂SiO)_{x]}R₂Si]*ₙ*-A

wherein
A is an aminofunctional endblocking group of the formula R^{A}CH₂CH(OH)CH₂OR²-
wherein R^{A} is an aminofunctional group, x is ≥ 0, m is from 2 to 4 inclusive, y is ≥ 4, *n* is ≥ 1,
R is independently a monovalent hydrocarbon group containing 1 to 30 carbons, a hydroxy group or an alkoxy group,
R¹ is a divalent hydrocarbon containing 2 to 30 carbons,
R² is a divalent hydrocarbon linking group containing 2 to 10, alternatively 2 to 6 carbon atoms,
wherein the composition provides protection. All subscripts and other groups such as R^{A}, R², x, m, y, n, R, R¹, and R² are as described above for the method of the invention.

When the composition is applied to hair, subjecting the composition to heat causes intermolecular reactions between silicone molecules, silicone molecules with the hair, or both between silicone molecules and silicone molecules and the hair.

Examples of additives which may be formulated into the compositions in addition to the silicone polyether emulsions include: additional silicones, anti-oxidants, cleansing agents, colorants, additional conditioning agents, deposition agents, electrolytes, emollients and oils, exfoliating agents, foam boosters, fragrances, humectants, occlusive agents, pediculicides, pH control agents, pigments, preservatives, biocides, other solvents, stabilizers, sun-screening agents, suspending agents, tanning agents, other surfactants, thickeners, vitamins, botanicals, fragrances, waxes, rheology-modifying agents, antidandruff, anti-acne, anti-carie and wound healing-promotion agents.

The composition, such as a shampoo or cleanser may contain at least one anionic detersive surfactant. This can be any of the well-known anionic detersive surfactants typically used in shampoo formulations. These anionic detersive surfactants function as cleansing agents and foaming agents in the shampoo compositions of this disclosure. The anionic detersive surfactants are exemplified by alkali metal sulforicinates, sulfonated glyceryl esters of fatty acids such as sulfonated monoglycerides of coconut oil acids, salts of sulfonated monovalent alcohol esters such as sodium oleylisethianate, amides of amino sulfonic acids such as the sodium salt of oleyl methyl tauride, sulfonated products of fatty acids nitriles such as palmitonitrile sulfonate, sulfonated aromatic hydrocarbons such as sodium alpha-naphthalene monosulfonate, condensation products of naphthalene sulfonic acids with formaldehyde, sodium octahydroanthracene sulfonate, alkali metal alkyl sulfates such as sodium lauryl sulfate, ammonium lauryl sulfate or triethanol amine lauryl sulfate, ether sulfates having alkyl groups of 8 or more carbon atoms such as sodium lauryl ether sulfate, ammonium lauryl ether sulfate, sodium alkyl aryl ether sulfates, and ammonium alkyl aryl ether sulfates, alkylarylsulfonates having 1 or more alkyl groups of 8 or more carbon atoms, alkylbenzenesulfonic acid alkali metal salts exemplified by hexylbenzenesulfonic acid sodium salt, octylbenzenesulfonic acid sodium salt, decylbenzenesulfonic acid sodium salt, dodecylbenzenesulfonic acid sodium salt, cetylbenzenesulfonic acid sodium salt, and myristylbenzenesulfonic acid sodium salt, sulfuric esters of polyoxyethylene alkyl ether including CH₃(CH₂)₆CH₂O(C₂H₄O)₂SO₃H, CH₃(CH₂)₇CH₂O(C₂H₄O)_{3.5}SO₃H, CH₃(CH₂)₈CH₂O(C₂H₄O)₈SO₃H, CH₃(CH₂)₁₉CH₂O(C₂H₄O)₄SO₃H, and CH₃(CH₂)₁₀CH₂O(C₂H₄O)₆SO₃H, sodium salts, potassium salts, and amine salts of alkylnaphthylsulfonic acid. Preferably the detersive surfactant is selected from the group consisting of sodium lauryl sulfate, ammonium lauryl sulfate, triethanolamine lauryl sulfate, sodium lauryl ether sulfate, and ammonium lauryl ether sulfate. The anionic detersive surfactant is present in the shampoo compositions of this invention in an amount from about 5 to 50 wt% and preferably about 5 to 25 wt% based on the total weight of the composition.

The composition may contain at least one cationic deposition aid, preferably a cationic deposition polymer. The cationic deposition aid will generally be present at levels of from 0.001 to 5%, preferably from about 0.01 to 1%, more preferably from about 0.02% to about 0.5% by weight. The polymer may be a homopolymer or be formed from two or more types of monomers. The molecular weight of the polymer will generally be between 5 000 and 10 000 000, typically at least 10 000 and preferably in the range 100 000 to about 2 000 000. The polymers will have cationic nitrogen containing groups such as quaternary ammonium or protonated amino groups, or a mixture thereof. The cationic charge density has been found to need to be at least 0.1 meq/g, preferably above 0.8 or higher. The cationic charge density should not exceed 4 meq/g, it is preferably less than 3 and more preferably less than 2 meq/g. The charge density can be measured using the Kjeldahl method and should be within the above limits at the desired pH of use, which will in general be from about 3 to 9 and preferably between 4 and 8. The cationic nitrogen-containing group will generally be present as a substituent on a fraction of the total monomer units of the cationic deposition polymer. Thus when the polymer is not a homopolymer it can contain spacer noncationic monomer units. Such polymers are described in the CTFA Cosmetic Ingredient Directory, 3rd edition. Suitable cationic deposition aids include, for example, copolymers of vinyl monomers having cationic amine or quaternary ammonium functionalities with water soluble spacer monomers such as (meth)acrylamide, alkyl and dialkyl (meth)acrylamides, alkyl (meth)acrylate, vinyl caprolactone and vinyl pyrrolidine. The alkyl and dialkyl substituted monomers preferably have C1-C7 alkyl groups, more preferably C1-3 alkyl groups. Other suitable spacers include vinyl esters, vinyl alcohol, maleic anhydride, propylene glycol and ethylene glycol. The cationic amines can be primary, secondary or tertiary amines, depending upon the particular species and the pH of the composition. In general secondary and tertiary amines, especially tertiary, are preferred. Amine substituted vinyl monomers and amines can be polymerized in the amine form and then converted to ammonium by quaternization. Suitable cationic amino and quaternary ammonium monomers include, for example, vinyl compounds substituted with dialkyl aminoalkyl acrylate, dialkylamino alkylmethacrylate, monoalkylaminoalkyl acrylate, monoalkylaminoalkyl methacrylate, trialkyl methacryloxyalkyl ammonium salt, trialkyl acryloxyalkyl ammonium salt, diallyl quaternary ammonium salts, and vinyl quaternary ammonium monomers having cyclic cationic nitrogen-containing rings such as pyridinium, imidazolium, and quaternized pyrrolidine, e.g., alkyl vinyl imidazolium, and quaternized pyrrolidine, e.g., alkyl vinyl imidazolium, alkyl vinyl pyridinium, alkyl vinyl pyrrolidine salts. The alkyl portions of these monomers are preferably lower alkyls such as the C,-C., alkyls, more preferably C, and C2 alkyls. Suitable amine-substituted vinyl monomers for use herein include dialkylaminoalkyl acrylate, dialkylaminoalkyl methacrylate, dialkylaminoalkyl acrylamide, and dialkylaminoalkyl methacrylamide. The cationic deposition aids can comprise mixtures of monomer units derived from amine- and/or quaternary ammonium-substituted monomer and/or compatible spacer monomers. Suitable cationic deposition aids include, for example: copolymers of 1-vinyl-2-pyrrolidine and 1-vinyl-3-methylimidazolium salt (e.g., Chloride salt) (referred to in the industry by the Cosmetic, Toiletry, and Fragrance Association, "CTFA". as Polyquaternium-16) such as those commercially available from BASF Wyandotte Corp. (Parsippany, NJ, USA) under the LUVIQUAT tradename (e.g., LUVIQUAT FC 370); copolymers of 1-vinyl-2-pyrrolidine and dimethylaminoethyl methacrylate (referred to in the industry by CTFA as Polyquaternium-11) such as those commercially from Gar Corporation (Wayne, NJ, USA) under the GAFQUAT tradename (e.g., GAFQUAT 755N); cationic diallyl quaternary ammonium-containing polymer including, for example, dimethyldiallyammonium chloride homopolymer and copolymers of acrylamide and dimethy1diallyammonium chloride, referred to in the industry (CTFA) as Polyquaternium 6 and Polyquaternium 7, respectively; mineral acid salts of aminoalkyl esters of homo-and co-polymers of unsaturated carboxylic acids having from 3 to 5 carbon atoms, as described in U.S. Patent 4,009,256; and cationic polyacrylamides as described in our copending UK Application No. 9403156.4 (WO95/22311). Other cationic deposition aids that can be used include polysaccharide polymers, such as cationic cellulose derivatives and cationic starch derivatives. Cationic polysaccharide polymer materials suitable for use in compositions of the disclosure include those of the formula: A-O(R-N⁺R¹R²R³X⁻) wherein: A is an anhydroglucose residual group, such as starch or cellulose anhydroglucose residual, R is an alkylene oxyalklene, polyoxyalkylene, or hydroxyalkylene group, or combination thereof, R', R∼' and R3 independently are alkyl, aryl, alkylaryl, arylalkyl, alkoxyalkyl, or alkoxyaryl groups, each group containing up to about 18 carbon atoms, and the total number of carbon atoms for each cationic moiety (i.e., the sum of carbon atoms in R', R 2 and R') preferably being about 20 or less, and X is an anionic counterion , as previously described. Cationic cellulose is available from Amerchol Corp. (Edison, NJ, USA) in their Polymer iR (trade mark) and LR (trade mark) series of polymers, as salts of hydroxyethyl cellulose reacted with trimethyl ammonium substituted epoxide, referred to in the industry (CTFA) as Polyquaternium 10. Another type of cationic cellulose includes the polymeric quaternary ammonium salts of hydroxyethyl cellulose reacted with lauryl dimethyl ammonium-substituted epoxide, referred to in the industry (CTFA) as Polyquaternium 24. These materials are available from Amerchol Corp. (Edison, NJ, USA) under the tradename Polymer LM-200. Other cationic deposition aids that can be used include cationic guar gum derivatives, such as guar hydroxypropyltrimonium chloride (Commercially available from Celanese Corp. in their Jaguar trademark series). Other materials include quaternary nitrogen-containing cellulose ethers (e.g., as described in U.S. Patent 3,962,418), and copolymers of etherified cellulose and starch (e.g., as described in U.S. Patent 3,958,581).

The composition may contain a foam boosting agent. A foam booster is an agent which increases the amount of foam available from a system at a constant molar concentration of surfactant, in contrast to a foam stabilizer which delays the collapse of a foam. Foam building is provided by adding to the aqueous media an effective amount of a foam boosting agent. The foam boosting agent is preferably selected from the group consisting of fatty acid alkanolamides and amine oxides. The fatty acid alkanolamides are exemplified by isostearic acid diethanolamide, lauric acid diethanolamide, capric acid diethanolamide, coconut fatty acid diethanolamide, linoleic acid diethanolamide, myristic acid diethanolamide, oleic acid diethanolamide, stearic acid diethanolamide, coconut fatty acid monoethanolamide, oleic acid monoisopropanolamide, and lauric acid monoisopropanolamide. The amine oxides are exemplified by N-cocodimethylamine oxide, N-lauryl dimethylamine oxide, N-myristyl dimethylamine oxide, N-stearyl dimethylamine oxide, N-cocamidopropyl dimethylamine oxide, N-tallowamidopropyl dimethylamine oxide, bis(2-hydroxyethyl) C12-15 alkoxypropylamine oxide. Preferably a foam booster is selected from the group consisting of lauric acid diethanolamide, N-lauryl dimethylamine oxide, coconut acid diethanolamide, myristic acid diethanolamide, and oleic acid diethanolamide. The foam boosting agent is preferably present in the shampoo compositions of this disclosure in an amount from about 1 to 15 wt% and more preferably about 2 to 10 wt% based on the total weight of the composition. The composition may further comprise a polyalkylene glycol to improve lather performance. Concentration of the polyalkylene glycol in the shampoo composition may range from about 0.01% to about 5%, preferably from about 0.05% to about 3%, and more preferably from about 0. 1% to about 2%, by weight of the composition. The optional polyalkylene glycols are characterized by the general formula: H(OCH2CHR)n-OH wherein R is selected from the group consisting of H, methyl, and mixtures thereof. When R is H, these materials are polymers of ethylene oxide, which are also known as polyethylene oxides, polyoxyethylenes, and polyethylene glycols. When R is methyl, these materials are polymers of propylene oxide, which are also known as polypropylene oxides, polyoxypropylenes, and polypropylene glycols. When R is methyl, it is also understood that various positional isomers of the resulting polymers can exist. In the above structure, n has an average value of from about 1500 to about 25,000, preferably from about 2500 to about 20,000, and more preferably from about 3500 to about 15,000. Polyethylene glycol polymers useful herein are PEG-2M wherein R equals H and n has an average value of about 2,000 (PEG-2M is also known as Polyox WSR9 N-10, which is available from Union Carbide and as PEG-2,000); PEG-5M wherein R equals H and n has an average value of about 5,000 (PEG-5M is also known as Polyox WSRO N-35 and Polyox WSRS N-80, both available from Union Carbide and as PEG-5,000 and Polyethylene Glycol 300,000); PEG-7M wherein R equals H and n has an average value of about 7,000 (PEG-7M is also known as Polyox WSRO N-750 available from Union Carbide); PEG-9M wherein R equals H and n has an average value of about 9,000 (PEG 9-M is also known as Polyox WSRS N-3333 available from Union Carbide); and PEG14 M wherein R equals H and n has an average value of about 14,000 (PEG-14M is also known as Polyox WSRO N-3000 available from Union Carbide). Other useful polymers include the polypropylene glycols and mixed polyethylene/polypropylene glycols.

The composition may contain a suspending agent at concentrations effective for suspending the preferred silicone conditioning agent, or other water-insoluble material, in dispersed form in the shampoo compositions. Such concentrations range from about 0.1% to about 10%, preferably from about 0.3% to about 5.0%, by weight of the shampoo compositions. Suspending agents include crystalline suspending agents which can be categorized as acyl derivatives, long chain amine oxides, and mixtures thereof, concentrations of which range from about 0.1% to about 5.0%, preferably from about 0.5% to about 3.0%, by weight of the shampoo compositions. These suspending agents are described in U.S. Patent 4.741,855. These preferred suspending agents include ethylene glycol esters of fatty acids preferably having from about 16 to about 22 carbon atoms. More preferred are the ethylene glycol stearates, both mono and distearate, but particularly the distearate containing less than about 7% of the mono stearate. Other suitable suspending agents include alkanol amides of fatty acids, preferably having from about 16 to about 22 carbon atoms, more preferably about 16 to 18 carbon atoms, preferred examples of which include stearic monoethanolamide, stearic diethanolamide, stearic monoisopropanolamide and stearic monoethanolamide stearate. Other long chain acyl derivatives include long chain esters of long chain fatty acids (e.g., stearyl stearate, cetyl palmitate, etc.); glyceryl esters (e.g., glyceryl distearate) and long chain esters of long chain alkanol amides (e.g., stearamide diethanolamide distearate, stearamide monoethanolamide stearate). Long chain acyl derivatives, ethylene glycol esters of long chain carboxylic acids, long chain amine oxides, and alkanol amides of long chain carboxylic acids in addition to the preferred materials listed above may be used as suspending agents. For example, it is contemplated that suspending agents with long chain hydrocarbyls having C8-C22 chains may be used. Other long chain acyl derivatives suitable for use as suspending agents include N,N-dihydrocarbyl amido benzoic acid and soluble salts thereof (e.g., Na, K), particularly N,N-di(hydrogenated) C16, C18 and tallow amido benzoic acid species of this family, which are commercially available from Stepan Company (Northfield, Illinois, USA). Examples of suitable long chain amine oxides for use as suspending agents include alkyl (C16-C22) dimethyl amine oxides, e.g., stearyl dimethyl amine oxide. Other suitable suspending agents include xanthan gum at concentrations ranging from about 0.3% to about 3%, preferably from about 0.4% to about 1.2%, by weight of the shampoo compositions. The use of xanthan gum as a suspending agent in silicone containing shampoo compositions is described, for example, in U.S. Patent 4,788,006, which description is incorporated herein by reference. Combinations of long chain acyl derivatives and xanthan gum may also be used as a suspending agent in the shampoo compositions. Such combinations are described in U.S. Patent 4,704,272. Other suitable suspending agents include carboxyvinyl polymers. Preferred among these polymers are the copolymers of acrylic acid crosslinked with polyallylsucrose as described in U.S. Patent 2,798,053. Examples of these polymers include Carbopol 934, 940, 941, and 956. available from B. F. Goodrich Company. Other suitable suspending agents include primary amines having a fatty alkyl moiety having at least about 16 carbon atoms, examples of which include palmitamine or stearamine, and secondary amines having two fatty alkyl moieties each having at least about 12 carbon atoms, examples of which include dipalmitoylamine or di(hydrogenated tallow)amine. Still other suitable suspending agents include di(hydrogenated tallow)phthalic acid amide, and crosslinked maleic anhydride-methyl vinyl ether copolymer. Other suitable suspending agents may be used in the shampoo compositions, including those that can impart a gel-like viscosity to the composition, such as water soluble or colloidally water soluble polymers like cellulose ethers (e.g., methylcellulose, hydroxybutyl methylcellulose, hyroxypropylcellulose, hydroxypropyl methylcellulose, hydroxyethyl ethylcellulose and hydroxyethylcellulose), guar gum, polyvinyl alcohol, polyvinyl pyrrolidone, hydroxypropyl guar gum, starch and starch derivatives, and other thickeners, viscosity modifiers, gelling agents, etc.

The composition may contain one or more water-soluble emollients including lower molecular weight aliphatic diols such as propylene glycol and butylene glycol; polyols such as glycerine and sorbitol; and polyoxyethylene polymers such as polyethylene glycol 200. The specific type and amount of water soluble emollient(s) employed will vary depending on the desired aesthetic characteristics of the composition, and is readily determined by one skilled in the art.

The composition may contain various oils. The term "oil" as used herein refers to any material which is substantially insoluble in water. When the composition is to be used in a cosmetic or personal care product, the product components must also be cosmetically acceptable or otherwise meet the conditions of the end use product. Suitable oil components include natural oils such as coconut oil; hydrocarbons such as mineral oil and hydrogenated polyisobutene; fatty alcohols such as octyldodecanol; esters such as C12 - C15 alkyl benzoate; diesters such as propylene dipelarganate; and triesters, such as glyceryl trioctanoate and silicones especially cyclomethicone and dimethicone and mixtures thereof. The composition also contains oils, preferably a mixture of low viscosity and high viscosity oils. Suitable low viscosity oils have a viscosity of 5 to 100 mPa.s at 25°C, and are generally esters having the structure RCO-OR' wherein RCO represents the carboxylic acid radical and wherein OR' is an alcohol residue. Examples of these low viscosity oils include isotridecyl isononanoate, PEG-4 diheptanoate, isostearyl neopentanoate, tridecyl neopentanoate, cetyl octanoate, cetyl palmitate, cetyl ricinoleate, cetyl stearate, cetyl myristate, coco-dicaprylate/caprate, decyl isostearate, isodecyl oleate, isodecyl neopentanoate, isohexyl neopentanoate, octyl palmitate, dioctyl malate, tridecyl octanoate, myristyl myristate, octododecanol, or mixtures of octyldodecanol, acetylated lanolin alcohol, cetyl acetate, isododecanol, polyglyceryl-3-diisostearate, or mixtures thereof. The high viscosity surface oils generally have a viscosity of 200-1,000,000 mPa.s at 25°C, preferably a viscosity of 100,000-250,000 mPa.s. Surface oils include castor oil, lanolin and lanolin derivatives, triisocetyl citrate, sorbitan sesquioleate, C10-18 triglycerides, caprylic/capric/triglycerides, coconut oil, corn oil, cottonseed oil, glyceryl triacetyl hydroxystearate, glyceryl triacetyl ricinoleate, glyceryl trioctanoate, hydrogenated castor oil, linseed oil, mink oil, olive oil, palm oil, illipe butter, rapeseed oil, soybean oil, sunflower seed oil, tallow, tricaprin, trihydroxystearin, triisostearin, trilaurin, trilinolein, trimyristin, triolein, tripalmitin, tristearin, walnut oil, wheat germ oil, cholesterol, or mixtures thereof. The suggested ratio of low viscosity to high viscosity oils in the oil phase is 1:15 to 15:1, preferably 1:10 to 10:1 respectively. The preferred formulation of the invention comprises 1 to 20% of a mixture of low viscosity and high viscosity surface oils.

Mention may be made, among the optional other non-silicone fatty substances, of mineral oils, such as liquid paraffin or liquid petroleum, of animal oils, such as perhydrosqualene or arara oil, or alternatively of vegetable oils, such as sweet almond, calophyllum, palm, castor, avocado, jojaba, olive or cereal germ oil. It is also possible to use esters of lanolic acid, of oleic acid, of lauric acid, of stearic acid or of myristic acid, for example; alcohols, such as oleyl alcohol, linoleyl or linolenyl alcohol, isostearyl alcohol or octyldodecanol; or acetylglycerides, octanoates, decanoates or ricinoleates of alcohols or of polyalcohols. It is alternatively possible to use hydrogenated oils which are solid at 25°C, such as hydrogenated castor, palm or coconut oils, or hydrogenated tallow; mono-, di-, tri- or sucroglycerides; lanolins; or fatty esters which are solid at 25°C.

The composition may contain various waxes. The waxes or wax-like materials generally have a melting point range of 35 to 120°C at atmospheric pressure. Waxes in this category include synthetic wax, ceresin, paraffin, ozokerite, illipe butter, beeswax, carnauba, microcrystalline, lanolin, lanolin derivatives, candelilla, cocoa butter, shellac wax, spermaceti, bran wax, capok wax, sugar cane wax, montan wax, whale wax, bayberry wax, or mixtures thereof. The preferred formulation comprises about 10-30% of a mixture of waxes. Mention may be made, among the waxes capable of being used as non-silicone fatty substances, of animal waxes, such as beeswax; vegetable waxes, such as carnauba, candelilla, ouricury or japan wax or cork fibre or sugarcane waxes; mineral waxes, for example paraffin or lignite wax or microcrystalline waxes or ozokerites; synthetic waxes, including polyethylene waxes, and waxes obtained by the Fischer-Tropsch synthesis. Mention may be made, among the silicone waxes, of polymethylsiloxane alkyls, alkoxys and/or esters.

Thickening agent may be added to provide a convenient viscosity. For example, viscosities within the range of 500 to 25,000 mm²/s at 25°C or more alternatively in the range of 3,000 to 7,000 mm²/s are usually suitable. Suitable thickening agents are exemplified by sodium alginate, gum arabic, polyoxyethylene, guar gum, hydroxypropyl guar gum, ethoxylated alcohols, such as laureth-4 or polyethylene glycol 400, cellulose derivatives exemplified by methylcellulose, methylhydroxypropylcellulose, hydroxypropylcellulose, polypropylhydroxyethylcellulose, starch, and starch derivatives exemplified by hydroxyethylamylose and starch amylose, locust bean gum, electrolytes exemplified by sodium chloride and ammonium chloride, and saccharides such as fructose and glucose, and derivatives of saccharides such as PEG-120 methyl glucose diolate or mixtures of 2 or more of these. Alternatively the thickening agent is selected from cellulose derivatives, saccharide derivatives, and electrolytes, or from a combination of two or more of the above thickening agents exemplified by a combination of a cellulose derivative and any electrolyte, and a starch derivative and any electrolyte. The thickening agent, where used is present in the shampoo compositions of this disclosure in an amount sufficient to provide a viscosity in the final shampoo composition of from 500 to 25,000 mm²/s. Alternatively the thickening agent is present in an amount from about 0.05 to 10 wt% and alternatively 0.05 to 5 wt% based on the total weight of the composition.

Stabilizing agents can be used in the water phase of the compositions. Suitable water phase stabilizing agents can include alone or in combination one or more electrolytes, polyols, alcohols such as ethyl alcohol, and hydrocolloids. Typical electrolytes are alkali metal salts and alkaline earth salts, especially the chloride, borate, citrate, and sulfate salts of sodium, potassium, calcium and magnesium, as well as aluminum chlorohydrate, and polyelectrolytes, especially hyaluronic acid and sodium hyaluronate. When the stabilizing agent is, or includes, an electrolyte, it amounts to about 0.1 to 5 wt % and more alternatively 0.5 to 3 wt % of the total composition. The hydrocolloids include gums, such as Xantham gum or Veegum and thickening agents, such as carboxymethyl cellulose. Polyols, such as glycerine, glycols, and sorbitols can also be used. Alternative polyols are glycerine, propylene glycol, sorbitol and butylene glycol. If a large amount of a polyol is used, one need not add the electrolyte. However, it is typical to use a combination of an electrolyte, a polyol and an hydrocolloid to stabilize the water phase, e.g. magnesium sulfate, butylene glycol and Xantham gum.

The composition can also be under the form of aerosols in combination with propellant gases, such as carbon dioxide, nitrogen, nitrous oxide, volatile hydrocarbons such as butane, isobutane, or propane and chlorinated or fluorinated hydrocarbons such as dichlorodifluoromethane and dichlorotetrafluoroethane or dimethylether.

Silicone compositions other than the silicone compositions described above may also be included in the compositions. For example, such silicones include; silicone fluids, gums, resins, elastomers; silicone surfactants and emulsifiers such as silicone polyethers, organofunctional silicones such as aminofunctional silicones and alkylmethylsiloxanes.

Alkylmethylsiloxanes may be included in the present compositions. These siloxane polymers generally will have the formula Me₃SiO[Me₂SiO]_{y}[MeRSiO]_{z}SiMe₃, in which R is a hydrocarbon group containing 6-30 carbon atoms, Me represents methyl, and the degree of polymerization (DP), i.e., the sum of y and z is 3-50. Both the volatile and liquid species of alkymethysiloxanes can be used in the composition.

Silicone gums may be included in the present compositions. Polydiorganosiloxane gums are known in the art and are available commercially. They consist of generally insoluble polydiorganosiloxanes having a viscosity in excess of 1,000,000 centistoke (mm²/s) at 25 °C., alternatively greater than 5,000,000 centistoke (mm²/s) at 25 °C. These silicone gums are typically sold as compositions already dispersed in a suitable solvent to facilitate their handling. Ultra-high viscosity silicones can also be included as optional ingredients. These ultra-high viscosity silicones typically have a kinematic viscosity greater than 5 million centistoke (mm²/s) at 25 °C, to about 20 million centistoke (mm²/s) at 25 °C. Compositions of this type in the form of suspensions are most preferred, and are described for example in US Patent 6.013,682 (January 11, 2000).

Silicone resins may be included in the present compositions. These resin compositions are generally highly crosslinked polymeric siloxanes. Crosslinking is obtained by incorporating trifunctional and/or tetrafunctional silanes with the monofunctional silane and/or difunctional silane monomers used during manufacture. The degree of crosslinking required to obtain a suitable silicone resin will vary according to the specifics of the silane monomer units incorporated during manufacture of the silicone resin. In general, any silicone having a sufficient level of trifunctional and tetrafunctional siloxane monomer units, and hence possessing sufficient levels of crosslinking to dry down to a rigid or a hard film can be considered to be suitable for use as the silicone resin. Commercially available silicone resins suitable for applications herein are generally supplied in an unhardened form in low viscosity volatile or nonvolatile silicone fluids. The silicone resins should be incorporated into compositions of the disclosure in their non-hardened forms rather than as hardened resinous structures.

Silicone carbinol fluids may be included in the present compositions. These materials are described in WO 03/101412 A2, and can be commonly described as substituted hydrocarbyl functional siloxane fluids or resins.

Water soluble or water dispersible silicone polyether compositions may be included in the present compositions: These are also known as polyalkylene oxide silicone copolymers, silicone poly(oxyalkylene) copolymers, silicone glycol copolymers, or silicone surfactants. These can be linear rake or graft type materials, or ABA type where the B is the siloxane polymer block, and the A is the poly(oxyalkylene) group. The poly(oxyalkylene) group can consist of polyethylene oxide, polypropylene oxide, or mixed polyethylene oxide/polypropylene oxide groups. Other oxides, such as butylene oxide or phenylene oxide are also possible.

Compositions disclosed herein can be used in w/o, w/s, or multiple phase emulsions using silicone emulsifiers. Typically the water-in-silicone emulsifier in such formulation is non-ionic and is selected from polyoxyalkylene-substituted silicones, silicone alkanolamides, silicone esters and silicone glycosides. Suitable silicone-based surfactants are well known in the art, and have been described for example in US 4,122,029 (Gee et al.), US 5,387,417 (Rentsch), and US 5,811,487 (Schulz et al).

The compositions disclosed herein may be used on any hair type including Caucasian, African, Asian, and on chemically treated hair including colored, permed, relaxed, and bleached hair.

### Examples

All measurements and experiments were conducted at 23°C, unless indicated otherwise.

### Example 1

Synthesis of an epoxy terminal (AB)n silicone polyether copolymer:

To a 1 L flask was added 408.18 g of a 35 DP Si-H terminal polydimethylsiloxane, 0.4825 g of SYL-OFF 4000 (0.52 wt% Pt), and 58.16 g of 2-propanol. The headspace was purged with N₂ and the reactants were heated to 70 °C. 79.82 g of NOF Uniox DMUS-5 [dimethallyl terminal poly(ethylene oxide)] was added dropwise. Upon observation of a mild exotherm (∼2 °C), the temperature was increased to 90 °C during the addition of the polyether. Upon complete addition of the poly(ethylene oxide) (-20 minutes), the reaction mixture was allowed to heat for 1 hour. To this, 13.04 g of allyl glycidyl ether was added dropwise. The reaction was heated an additional 3 hours at 90 °C. Upon completion, the volatiles were removed in vacuo. Analysis of the polymer by ²⁹Si NMR suggests a value of n equal to 2.4 resulting in a calculated molecular weight of 11,300 g/mol corresponding to an average structure of:

CH₂(O)CHCH₂OCH₂CH₂CH₂[SiMe₂(OSiMe₂)₃₅OSiMe₂CH₂CH(CH₃)CH₂O(CH₂CH₂O) ₁₄CH₂CH(CH₃)CH₂]_{2.4}SiMe₂O(SiMe₂O)₃₅OSiMe₂CH₂CH₂CH₂OCH₂CH(O)CH₂.

Synthesis of an amine terminal (AB)n silicone polyether copolymer:

To a 250 mL flask was added 100.03 g of the above epoxy terminal (AB)n silicone polyether copolymer, 2.94 g of diisopropanolamine, and 24.87 g of 2-propanol. The headspace was purged with N₂ and the mixture was heated at reflux for 4 hours. Volatiles were removed in vacuo. Analysis by ¹³C NMR confirms disappearance of the epoxy moiety due to reaction with the amine. In terms of composition based on the functional portions, the polymer is 81 wt% silicone, 13 wt% ethylene oxide, and 0.49 wt% nitrogen.

### Example 2

### Preparation of Emulsion A:

The emulsification procedure was on performed on a Hauschild Speedmixer (Model DAC 150 FVZ). To a plastic 20 g size cup was added 10.18g of the silicone copolymer from Example 1 along with 0.53 g of Brig 35L surfactant and 3.12 g of water. The mixture was sheared at maximum speed for 20 seconds. The gel-like phase and particles were worked with a spatula to aid incorporation of the water phase. The shear step and subsequent working with the spatula were repeated until all of the water was incorporated to create a homogeneous mixture. A final addition of 3.00 g of water was made and the emulsion of was again mixed on the Speedmixer.

### Example 3

### Preparation of Emulsion B:

To a 20 g cup was added 4.1 g of the silicone copolymer described in Example 1. To the vessel, 0.099 g of lactic acid, 4.0 g of Brij 35L, and 2.2 g of water was added. The mixture was sheared for 20 seconds at maximum speed on the Speedmixer. Aliquots of 1-3 g of water were added until a total of 10.1 g had been added. The mixture was sheared on the Speedmixer after each addition and worked with a spatula to aid incorporation of the water into the gel-like phase.

### Example 4

### Preparation of Emulsion C:

To a plastic 20 g size cup was added 10.20g of an (AB)n silicone polyether copolymer synthesized in an analogous manner as Example 1 (MW = 25,300; 91 wt% silicone, 5.8 wt% ethylene oxide, 0.22 wt% nitrogen extrapolated from analysis by NMR). To the cup was also added 0.60 g of Brig 35L surfactant and 2.30 g of water. The mixture was sheared at maximum speed for 20 seconds. The gel-like phase and particles were worked with a spatula to aid incorporation of the water phase. The shear step and subsequent working with the spatula were repeated until all of the water was incorporated to create a homogeneous mixture. Further additions of 1.3, 2.1, and 0.80 g of water were made with shearing in the Speedmixer.

### Example 5

### Preparation of Emulsion D:

To a 20 g Speedmixer cup was added 4.2 g of the (AB)n silicone polyether copolymer described in Example 4. To the vessel was also added 0.063 g of lactic acid, 4.2 g of Brij 35L, and 2.3 g of water. The mixture was sheared for 20 seconds at maximum speed on the Speedmixer. Aliquots of 1-3 g of water were added until a total of 9.5 g had been added. The mixture was sheared on the Speedmixer after each addition and worked with a spatula to aid incorporation of the water into the gel-like phase.

### Example 6

### Preparation of Emulsion E:

To a 100 g Speedmixer cup was added 20.0 g of the (AB)n silicone polyether copolymer (MW = 30,100; 87 wt% silicone, 11 wt% ethylene oxide, 0.19 wt% nitrogen extrapolated from analysis by NMR). To the vessel was also added 0.963 g of lactic acid, 20.8 g of Brij 35L, and 9.1 g of water. The mixture was sheared for 30 seconds at maximum speed on the Speedmixer. Aliquots of 8-12 g of water were added until a total of 50.4 g had been added. The mixture was sheared on the Speedmixer after each addition and worked with a spatula to aid incorporation of the water into the gel-like phase.

### Example 7

### Preparation of Emulsion F:

To a 100 g Speedmixer cup was added 20.2 g of the (AB)n silicone polyether copolymer (MW = 38,400; 93 wt% silicone, 4.9 wt% ethylene oxide, 0.15 wt% nitrogen extrapolated from analysis by NMR). To the vessel was also added 0.128 g of lactic acid, 20.2 g of Brij 35L, and 9.5 g of water. The mixture was sheared for 30 seconds at maximum speed on the Speedmixer. Aliquots of 7-12 g of water were added until a total of 49.6 g had been added. The mixture was sheared on the Speedmixer after each addition and worked with a spatula to aid incorporation of the water into the gel-like phase.

### Example 8

### Preparation of Emulsion G:

To a 250 mL stainless steel beaker was added 20.19 g of the silicone polyether copolymer described in Example 7 along with 6.67 g of Tergitol 15-S-12 and 3.34 g of Tergitol 15-S-5. The components were mixed at 500 rpm for five minutes using a Cowles blade affixed to a Caframo mixer (type RZR50). After addition of 0.5 mL of acetic acid, mixing was resumed for an additional 5 minutes. During mixing, 10.10 g of DI water was added slowly and shearing was allowed to continue for 10 minutes. An additional aliquot of DI water, 10.24 g, was added and mixing continued for 30 minutes. Another portion of DI water, 10.19 g was added and followed by continued mixing over 30 minutes. The mixer was stopped occasionally and the walls were scraped down to ensure full incorporation of the polymer into the water phase. A final addition of 29.57 g of DI water was made and the emulsion was mixed for 3 hours. After collecting the emulsion, 9.90 g of DI water were added to adjust the actives content to the desired level.

### Example 9

Synthesis of a chain-extended amino functional (AB)n silicone polyether copolymer:

To a 2 L flask was added 131.7 g of NOF DMUS-5 [dimethallyl terminal poly(ethylene oxide)], 1.76 g of a Pt solution (0.33 wt% Pt in IPA, diluted Dow Corning 2-0719 catalyst), and 111.2 g of ethylacetate. The headspace was purged with N₂ and the reactants were heated to 70 °C. To the flask was added 868.5 g of a 42 DP Si-H terminal polydimethylsiloxane dropwise over 30 minutes. Upon observation of a mild exotherm (∼2 °C) change in color of the reaction mixture to light yellow, the temperature was increased to 90 °C during the addition of the siloxane polymer. Upon complete addition of the siloxane polymer, the reaction mixture was allowed to heat for 2 hours wherein 24.31 g of allyl glycidyl ether was then added. The reaction was allowed to heat for 4 hours at 100 °C. Upon completion, the volatiles were removed in vacuo. Analysis of the polymer by ²⁹Si NMR suggests a value of n equal to 1.5 resulting in a calculated molecular weight of 9,600 g/mol corresponding to an average structure of:

CH₂(O)CHCH₂OCH₂CH₂CH₂[SiMe₂(OSiMe₂)₄₂OSiMe₂CH₂CH(CH₃)CH₂O(CH₂CH₂O) ₁₄CH₂CH(CH₃)CH₂]₁.₅SiMe₂O(SiMe₂O)₄₂OSiMe₂CH₂CH₂CH₂OCH₂CH(O)CH₂.

To a 1 L flask was added 444.7 g of the epoxy terminal (AB)n silicone copolymer, 5.38 g of piperazine, and 151.1 g of 2-propanol. The contents were heated at reflux for 12 hours.

The volatile components were then removed in vacuo. Analysis by ¹³C and ²⁹Si NMR spectroscopy suggests a molecular weight of 23,400 g/mol was obtained with a composition of the amino functional chain-extended polymer of 82 wt% silicone, 9.3 wt% ethylene oxide, and 0.41 wt% nitrogen.

### Example 10

### Preparation of Emulsion H:

To a 100 g Speedmixer cup was added 59.99 g of the chain-extended amino functional (AB)n silicone polyether copolymer described in Example 9. To the vessel was also added 0.21 g of lactic acid, 3.38 g of Brij 35L, and 15.1 g of DI water. The mixture was sheared for 30 seconds at maximum speed on the Speedmixer. Aliquots of 5-7 g of water were added until a total of 22.04 g had been added. The mixture was sheared on the Speedmixer after each addition and worked with a spatula to aid incorporation of the water into the gel-like phase.

### Example 11

### Preparation of Emulsion I:

To a 100 g Speedmixer cup was added 60.00 g of an alternative chain-extended amino functional (AB)n silicone polyether copolymer (MW = 23,900; 92 wt% silicone, 3.5 wt% ethylene oxide, 0.26 wt% nitrogen extrapolated from analysis by NMR) synthesized in an analogous manner to Example 9. To the vessel was also added 0.13 g of lactic acid, 3.88 g of Brij 35L, and 15.1 g of DI water. The mixture was sheared for 30 seconds at maximum speed on the Speedmixer. Aliquots of 5-11 g of water were added until a total of 22.1 g had been added. The mixture was sheared on the Speedmixer after each addition and worked with a spatula to aid incorporation of the water into the gel-like phase.

**Table 1 - Emulsion Compositions**

| **Emulsion ID** | **Example #** | % **Silicone Polymer** | **D₅₀ Particle Size (µm)** |
|---|---|---|---|
| A | 2 | 60.5 | 0.69 |
| B | 3 | 20.1 | 0.16 |
| C | 4 | 59.0 | 2.39 |
| D | 5 | 20.8 | 0.22 |
| E | 6 | 19.7 | 1.57 |
| F | 7 | 20.3 | 0.23 |
| G | 8 | 20.1 | 0.04 |
| H | 10 | 59.6 | 0.54 |
| I | 11 | 59.3 | 1.56 |

### Test Procedure

Test protocols close to consumer habits were developed to reproducibly treat and heat damage slightly bleached Caucasian hair. Tresses treated with different silicones were consistently exposed for 2 min into a calibrated oven from 130 up to 240°C and compared to untreated tresses and/or control formulation. Mechanical resistance of heat damaged hair fibers was evaluated using hand combing test to quantify the weight of broken fibers of wet tresses. Results show a significant reduction of broken hair when hair is treated with the silicone polyether copolymer of the present invention.

### EXAMPLES 13, 15-16

Silicone materials were evaluated according to the following procedure. To 4 g of wet virgin Latino curly hair tresses was applied 100 µl per gram of hair of 3% (w/w) active solution in water (emulsion) or cyclopentasiloxane (polymer). The tresses were then dried at 70 °C for 20 minutes. After drying, the tresses were placed in an over at 130 to 240 °C for 2 minutes. The tresses were then removed from the oven and detangled using a comb until no broken hair is collected. The weight of the dried broken hair collected from the treated tresses was then compared to that of untreated control tresses and the percent improvement calculated. The results of testing of several silicone compositions is in the table below.

| Example | Silicone Name | Description | Improvement (%) (neat)** | Improvement (%) (cream)⁺ | Active Silicone % (w/w) |
|---|---|---|---|---|---|
| 13 | Bis-Diisopropanolam ino-PG-propyl | Aminosiloxane polyether (AB)ₙ nonionic | 80 | 82 | 56 |
| | Dimethicone / Bis-Isobutyl PEG-14 Copolymer (and) Polysorbate 20 (and) Butyloctanol | emulsion (Dow Corning® CE-8411) | | | |
| 15 | PEG-7 Dimethicone (and). Laureth-7 (and) Polysorbate 20 | Silicone polyether microemulsion (Dow Corning® CE-1874) | 60 | NA | 30 |
| 16* | C11-13 Isoparaffin (and) Dimethiconol (and) Isohexadecane (and) Dimethicone | PDMS gum (Xiameter® PMX-1502 Fluid) | 33 | 21 | NA |

| | | | | | |
|---|---|---|---|---|---|
| * 3% (w/w) Neat material on bleached hair. + 3% (w/w) in Leave-on cream on bleached hair. | | | | | |

The following is an example of the leave-on cream formulation used in examples 13, 15-16:

| Part | INCI Name | % (w/w) |
|---|---|---|
| A | Water | q.s |
| | Hydroxyethylcellulose | 1.5 |
| | Glycerin | 5 |
| B | Behentrimonium Methosulfate (and) Cetearyl Alcohol | 3 |
| | C10-40 Isoalkylamidopropylethyldimonium Ethosulfate and Dipropylene Glycol | 2.5 |
| | Jojoba Oil | 1 |
| | Cetyl Alcohol | 0.3 |
| C | Hydrolyzed Wheat Gluten | 0.05 |
| | Panthenol | |
| | Bis-diisopropanolamino-PG-propyl Dimethicone / Bis-isobutyl PEG-14 Copolymer (and) polysorbate 20 (and) butyloctanol | 5.36 |
| | Water (and) heliantus annus seed oil (and) glycerin (and) bambusa vulgaris extract (and) sclerotium gum (and) tocopherol | 1.0 |
| | DMDM Hydantoin | 0.1 |

## Claims

1. A method for protecting hair from heat, the method comprising applying to hair a composition comprising a silicone polyether copolymer having an average formula
A-R₂SiO(R₂SiO)ₓSiR₂-[[R¹O(CₘH₂ₘO)_{y}R¹][R₂SiO(R₂SiO)ₓ]R₂Si]*ₙ*-A
wherein
A is an aminofunctional endblocking group of the formula R^{A}CH₂CH(OH)CH₂OR²-wherein R^{A} is an aminofunctional group,
x is ≥ 0, m is from 2 to 4 inclusive, y is ≥ 4, *n* is ≥ 1,
R is independently a monovalent hydrocarbon group containing 1 to 30 carbons, a hydroxy group or an alkoxy group,
R¹ is a divalent hydrocarbon containing 2 to 30 carbons,
R² is a divalent hydrocarbon linking group containing 2 to 20 carbon atoms,
wherein the composition provides protection.

2. The method of claim 1, wherein R^{A} has a formula selected from H(R³)N- , (R³)₂N-, and (R³)₃N-, where R³ is independently a monovalent organic group containing 1 to 30 carbon atoms.

3. The method of claim 1, wherein R^{A} is (CH₃)HN-, (CH₃)₂N-, (CH₃CH₂)HN-, (CH₃CH₂)₂N-, (HOCH₂CH₂)₂N-, piperazine, or [CH₂CH(OH)CH₃]₂N-.

4. The method of any one of the preceding claims wherein the composition is an emulsion.

5. The method of any one of the preceding claims, wherein the treated hair is subjected to temperatures up to 240 °C.

6. The method of claim 5, wherein the heat causes intermolecular reactions between silicone molecules, silicone molecules with the hair, or both between silicone molecules and silicone molecules and the hair.

7. The method of any one of the preceding claims, wherein the composition is applied to the hair less than 30 minutes prior to application of the heat.

## Patentansprüche

1. Ein Verfahren zum Schutz des Haares vor Wärme, wobei das Verfahren das Aufbringen einer Zusammensetzung auf das Haar beinhaltet, die ein Silikonpolyether-Copolymer beinhaltet, das die folgende durchschnittliche Formel aufweist:
A-R₂SiO(R₂SiO)ₓSiR₂-[[R¹O(CₘH₂ₘO)_{y}R¹][R₂SiO(R₂SiO)_{x]}R₂Si]*ₙ*-A,
wobei
A eine aminofunktionelle Endblockierungsgruppe der Formel R^{A}CH₂CH(OH)CH₂OR²- ist,
wobei R^{A} eine aminofunktionelle Gruppe ist,
x ≥ 0 ist, m von 2 bis einschließlich 4 beträgt, y ≥ 4 ist, *n* ≥ 1 ist,
R unabhängig eine einwertige Kohlenwasserstoffgruppe ist, die 1 bis 30 Kohlenstoffe, eine Hydroxygruppe oder eine Alkoxygruppe enthält,
R¹ ein zweiwertiger Kohlenwasserstoff ist, der 2 bis 30 Kohlenstoffe enthält,
R² eine zweiwertige Kohlenwasserstoffverknüpfungsgruppe ist, die 2 bis 20 Kohlenstoffatome enthält,
wobei die Zusammensetzung Schutz bereitstellt.

2. Verfahren gemäß Anspruch 1, wobei R^{A} eine Formel, ausgewählt aus H(R³)N-, (R³)₂N-, und (R³)₃N- aufweist, wobei R³ unabhängig eine einwertige organische Gruppe ist, die 1 bis 30 Kohlenstoffatome enthält.

3. Verfahren gemäß Anspruch 1, wobei R^{A} (CH₃)HN-, (CH₃)₂N-, (CH₃CH₂)HN-, (CH₃CH₂)₂N-, (HOCH₂CH₂)₂N-, Piperazin oder [CH₂CH(OH)CH₃]₂N- ist.

4. Verfahren gemäß einem der vorhergehenden Ansprüche, wobei die Zusammensetzung eine Emulsion ist.

5. Verfahren gemäß einem der vorhergehenden Ansprüche, wobei das behandelte Haar Temperaturen bis zu 240 °C ausgesetzt wird.

6. Verfahren gemäß Anspruch 5, wobei die Wärme intermolekulare Reaktionen zwischen Silikonmolekülen, Silikonmolekülen mit dem Haar oder sowohl zwischen Silikonmolekülen als auch Silikonmolekülen und dem Haar hervorruft.

7. Verfahren gemäß einem der vorhergehenden Ansprüche, wobei die Zusammensetzung weniger als 30 Minuten vor dem Aufbringen der Wärme auf das Haar aufgebracht wird.

## Revendications

1. Une méthode pour protéger les cheveux de la chaleur, la méthode comprenant l'application, sur les cheveux, d'une composition comprenant un copolymère de silicone-polyéther ayant une formule moyenne
A-R₂SiO(R₂SiO)ₓSiR_{d}[R¹O(CₘH₂ₘO)_{y}R¹][R₂SiO(R₂SiO)ₓ]R₂Si]*ₙ*-A
dans laquelle
A est un groupe aminofonctionnel de blocage d'extrémité de la formule R^{A}CH₂CH(OH)CH₂OR²- dans laquelle R^{A} est un groupe aminofonctionnel,
x est ≥ 0, m vaut de 2 à 4 inclus, y est ≥ 4, *n* est ≥ 1,
R est indépendamment un groupe hydrocarboné monovalent contenant de 1 à 30 carbones, un groupe hydroxy ou un groupe alcoxy,
R¹ est un hydrocarbure divalent contenant de 2 à 30 carbones,
R² est un groupe de liaison d'hydrocarbure divalent contenant de 2 à 20 atomes de carbone,
dans laquelle la composition fournit la protection.

2. La méthode de la revendication 1, dans laquelle R^{A} a une formule sélectionnée parmi H(R³)N-, (R³)₂N-, et (R³)₃N-, où R³ est indépendamment un groupe organique monovalent contenant de 1 à 30 atomes de carbone.

3. La méthode de la revendication 1, dans laquelle R^{A} est (CH₃)HN-, (CH₃)₂N-, (CH₃CH₂)HN-, (CH₃CH₂)₂N-, (HOCH₂CH₂)₂N-, la pipérazine, ou [CH₂CH(OH)CH₃]₂N-.

4. La méthode de l'une quelconque des revendications précédentes dans laquelle la composition est une émulsion.

5. La méthode de l'une quelconque des revendications précédentes, dans laquelle les cheveux traités sont soumis à des températures allant jusqu'à 240 °C.

6. La méthode de la revendication 5, dans laquelle la chaleur entraîne des réactions intermoléculaires entre des molécules de silicone, des molécules de silicone avec les cheveux, ou aussi bien entre des molécules de silicone que des molécules de silicone et les cheveux.

7. La méthode de l'une quelconque des revendications précédentes, dans laquelle la composition est appliquée sur les cheveux moins de 30 minutes avant l'application de la chaleur.
